# EUROPÄISCHE PATENTSCHRIFT

(11) **EP 0 879 810 B1**
(45) Veröffentlichungstag und Bekanntmachung des Hinweises auf die Patenterteilung: **20.02.2002**
(21) Anmeldenummer: 98108389.2
(22) Anmeldetag: 08.05.1998
(51) Int. Cl.: C07C 29/58, C07C 31/02

(54) **Verfahren zur Hydrolyse von Alkylmonohalogeniden**
Process for the hydrolysis of alkylmonohalides
Procédé pour l'hydrolyse de monohalogénures d'alkyle

(30) Priorität: 21.05.1997 DE 19721301
(43) Veröffentlichungstag der Anmeldung: 25.11.1998
(73) Patentinhaber: BASF AKTIENGESELLSCHAFT, 67056 Ludwigshafen (DE)
(72) Erfinder: Stabel, Uwe, Dr., 67166 Otterstadt (DE); Fahrbach, Gerhard, Dr., 68723 Plankstadt (DE); Neumann, Werner, 67067 Ludwigshafen (DE)
(74) Vertreter: Isenbruck, Günter, Dr.

(56) Entgegenhaltungen:
- EP-A- 0 479 350
- EP-A- 0 694 328
- US-A- 5 490 919

## Beschreibung

Die Erfindung betrifft die Hydrolyse von Alkylmonohalogeniden aus einem diese enthaltenden Gemisch sowie eine Verwendung.

Alkylhalogenide entstehen in Prozeßgasen oder Nebenströmen bei Reaktionen, in denen Chlor, Chloride oder PVC eingesetzt werden, oder beispielsweise in Abgasen aus Kunststoffrecycling-Anlagen, die PVC-haltige Materialien verarbeiten. Wegen der toxischen Wirkung können die Organohalogen-Verbindungen nicht ohne weiteres in die Atmosphäre entlassen werden und für die Weiterverwertung der Gase oder Nebenströme beispielsweise als Brennstoff oder als Einsatzstoff für weitere chemische Prozesse wie Steamcracker oder in Raffinerien sind niedrige Werte an organischem Chlor entscheidend.

Es sind bereits eine Vielzahl von Verfahren zur Umwandlung von Organohalogen-Verbindungen in halogenfreie Kohlenwasserstoffe bekannt.

Die EP-A-726 306 beschreibt ein katalytisches Hydrierverfahren unter Zusatz von Wasserstoff für Gase, die Chlorkohlenwasserstoffe enthalten können, an Edelmetallkontakten, insbesondere Kobalt-Molybdän- oder Nickel-Molybdän-Katalysatoren.

Nachteilig ist das Katalysator-Standzeitproblem, die Gefahr der Vergiftung der aktiven Komponenten beispielsweise durch Schwefel, die Notwendigkeit, Wasserstoff zuzusetzen, sowie die Bildung von korrosivem Chlorwasserstoff.

Die US 5,490,919 beschreibt beispielsweise die Dehalogenierung von chlor-, fluor- oder bromhaltigen Organohalogeniden durch die Reaktion mit einem Alkalihydroxid, insbesondere Natrium- oder Kaliumhydroxid, in alkoholischer Lösung in Gegenwart katalytischer Mengen eines Dehydrier-Katalysators, beispielsweise Palladium/Kohlenstoff bei Temperaturen zwischen 50 und 150°C und Normaldruck. Nachteilig ist das Katalysator-Standzeitproblem wegen der Aktivkomponente Palladium sowie der Zusatz artfremder Alkohole als Lösungsmittel.

Die EP-A-0 694 328 beschreibt ein Verfahren zur Reinigung halogenhaltiger Abgase einer Müllverbrennungsanlage durch Absorption in einem Wanderbett aus Calciumoxid und/oder Calciumhydroxid, das 2 bis 20 Gew.-% Aktivkohle enthalten kann. Es wird ein Abscheidegrad für HCl von über 99% erreicht. Nachteilig ist insbesondere das Feststoffhandling. Abgase aus Müllverbrennungsanlagen enthalten Chlor überwiegend in Form von Chlorwasserstoff, die Wirksamkeit des beschriebenen Verfahrens für höhere Konzentrationen an Organohalogen-Verbindungen ist nicht nachgewiesen.

Der vorliegenden Erfindung liegt demgegenüber die Aufgabe zugrunde, ein Hydrolyseverfahren zur Verfügung zu stellen, das einfach und sicher arbeitet und hohe Umsetzungsgrade für monosubstituierte Alkylhalogenide auch in höheren Konzentrationen gewährleistet. Das erfindungsgemäße Verfahren soll mit technisch einfachen Prozeßapparaten, ohne Standzeitprobleme (Vergiftung), sowie mit einfach zu handhabenden und kostengünstigen Reagenzien auskommen. Die Entstehung von Chlorwasserstoff soll vermieden werden und somit der Einsatz von korrosionsbeständigen Sondermaterialien für die Prozessapparate. Alkylmonohalogenide aus beispielsweise Prozeßgasen sollen in nahezu quantitativer Ausbeute in die entsprechenden Alkohole übergeführt werden.

Die der Erfindung zugrunde liegende Aufgabe wird dadurch gelöst, daß die Hydrolyse des Alkylmonohalogenide enthaltenden Gemisches an mit Alkali- und/oder Erdalkalihydroxiden imprägnierter Aktivkohle in Gegenwart von Wasser durchgeführt wird.

Die mit Alkali- und/oder Erdalkalihydroxiden imprägnierte Aktivkohle fungiert als Hydrolyseaktivator, der die nahezu quantitative Umsetzung der Alkylmonohalogenide zu Alkoholen bewirkt.

Überraschenderweise zeigte sich, daß sich organische Halogenverbindungen an mit Alkali- und/oder Erdalkalihydroxiden imprägnierter Aktivkohle leicht hydrolysieren lassen.

Das erfindungsgemäße Verfahren läßt sich in besonders vorteilhafter Weise auf die niederen primären Alkylhalogenide, insbesondere auf C₁- bis C₄-Alkylmonohalogenide, vorzugsweise auf C₁- bis C₃-Alkylmonohalogenide bzw. auf Gemische anwenden, die C₁- bis C₄-Alkylmonohalogenide, vorzugsweise C₁- bis C₃- Alkylmonohalogenide enthalten.

Die thermisch besonders stabilen niederen primären organischen Chloride, wie Methylchlorid, Ethylchlorid und n-Propylchlorid werden nach dem erfindungsgemäßen Verfahren zu mindestens 99% zu dem entsprechenden Alkohol umgesetzt. Das Verfahren ist ebenso zur Hydrolyse der entsprechenden Bromide und Jodide geeignet, deren Umsetzung sich entsprechend einfacher gestaltet, da sie thermisch nicht so stabil sind.

Besonders vorteilhaft ist es, die Aktivkohle mit wäßrigen Lösungen von 1 bis 40 Gew.-%, insbesondere 5 bis 25 Gew.-% Alkali- und/oder Erdalkalihydroxid zu imprägnieren. In vorteilhafter Weise wird Natriumhydroxid verwendet.
Ein weiterer Vorteil der Erfindung ist, daß in einem weiten Temperaturbereich, von Raumtemperatur bis 600°C, gearbeitet werden kann, wobei der Bereich von 120 bis 300°C besonders vorteilhaft ist.

Die Hydrolyse kann in technisch vorteilhafter Weise drucklos, aber auch unter Unterdruck oder Überdruck durchgeführt werden, wobei ein Druckbereich von 0,5 bar a (bar absolut) bis 50 bar a, inbesondere von 1 bis 5 bar a, besonders vorteilhaft ist.

Besonders vorteilhaft ist weiterhin die Flexibilität des erfindungsgemäßen Verfahrens bezüglich der einzusetzenden Komponenten, die sowohl in Gasphase als auch in flüssiger Phase vorliegen können.

Darüber hinaus ist das erfindungsgemäße Verfahren auch dazu geeignet, ausgehend von entsprechend substituierten Kohlenwasserstoff-Halogeniden, die verschiedensten Alkohole großtechnisch auf einfache und kostengünstige Weise zugänglich zu machen.

Die Erfindung wird im folgenden anhand von Ausführungsbeispielen und einer Zeichnung, die das Verfahren schematisch zeigt, näher erläutert.

### Beispiel 1

Ein Prozeßgasstrom aus einer Kunststoffrecyclinganlage von 20 l/h mit einem Chlorgehalt von insgesamt 5000 mg/Nm3 insbesondere aus Methylchlorid, Ethylchlorid, n-Propylchlorid, n-Butylchlorid und Chlorbenzol wird über einen Wärmetauscher 1 auf 150°C erhitzt und durch einen 70 cm langen und 1 cm breiten Rohrreaktor 2 bei 150° und 0,1 bar a Austrittsdruck geleitet. Der Rohrreaktor 2 ist mit einem Hydrolyseaktivator gefüllt, der aus mit 10 Gew.-% Natriumhydroxid imprägnierter Aktivkohle der Korngröße 2 bis 5 mm besteht. Im Versuch wurde die Aktivkohle IVP4 der Firma Chemviron eingesetzt.

Die eingesetzten Halogenverbindungen werden mit nahezu quantitativer Ausbeute umgesetzt, wie aus der nachfolgenden Tabelle 1 ersichtlich:

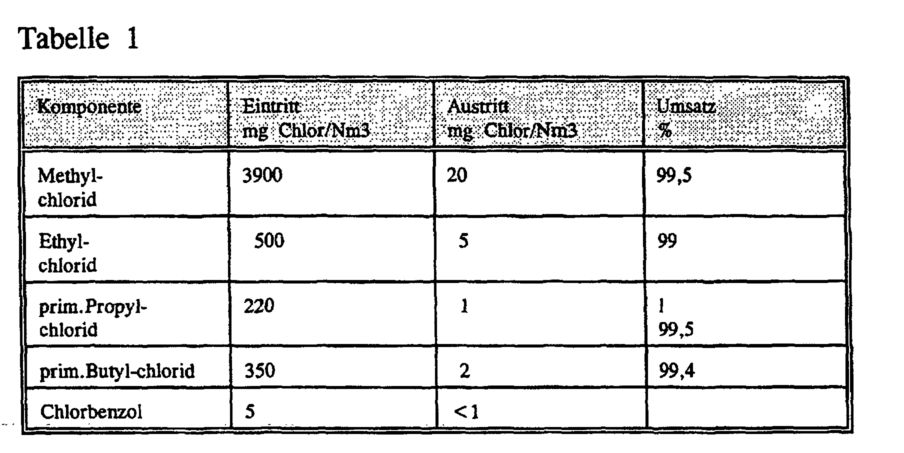

### Beispiel 2

Ein Prozeßgasstrom wie in Beispiel 1 beschrieben, wird unter denselben Bedingungen, mit Ausnahme der Reaktionstemperatur, die nun 250°C beträgt, mit den in Tabelle 2 aufgeführten Ausbeuten umgesetzt:

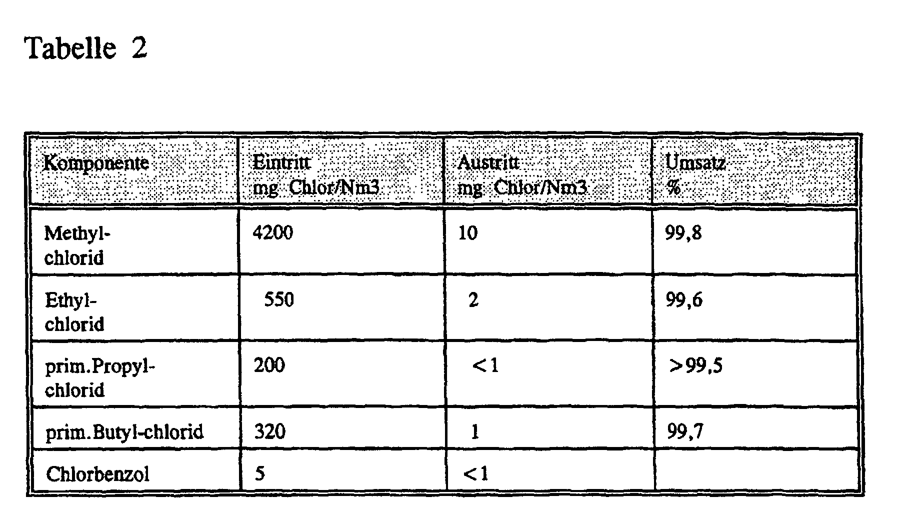

### Beispiel 3

Die Umsetzung wird unter denselben Bedingungen wie in Beispiel 1 durchgefiihrt, jedoch wird der Prozeßgasstrom durch einen Stickstoffstrom ersetzt und die Chlorverbindungen durch die entsprechenden Bromverbindungen. Die Ausbeuten sind aus Tabelle 3 ersichtlich.

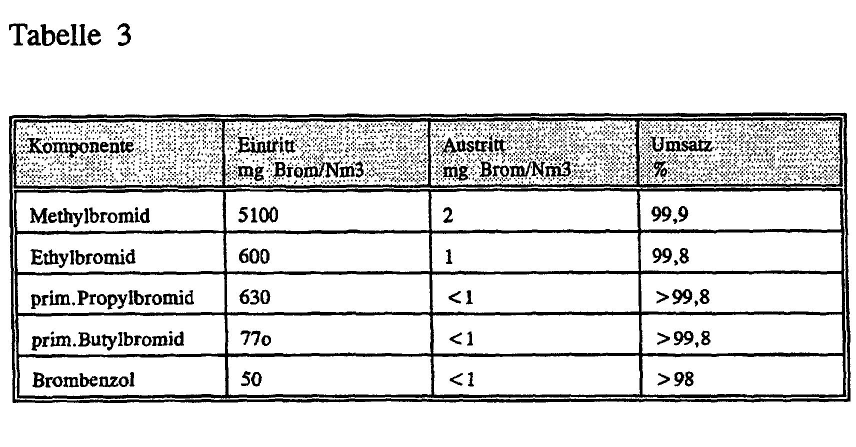

Der Kern der Erfindung besteht darin, daß Alkylmonohalogenide aus beispielsweise Prozeßgasen unter Zusatz von Wasser durch einen mit einem Hydrolyseaktivator gefüllten Reaktor geleitet werden, wobei als Hydrolyseaktivator mit Alkali- und/oder Erdalkalihydroxid imprägnierte Aktivkohle eingesetzt wird. Dabei reagieren die organischen Halogenide nahezu quantitativ zu den entsprechenden Alkoholen, wobei das Halogen als anorganisches Salz anfällt: 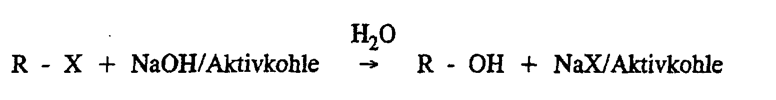 (X=Cl, Br, J)

Nachdem das Alkali- und/oder Erdalkalihydroxid verbraucht ist, wird der Hydrolyseaktivator durch Ausspülen der angereicherten anorganischen Halogenide beispielsweise mit Wasser gereinigt und erneut mit Alkali- und/oder Erdalkalihydroxidlösung aktiviert und wieder eingesetzt. Es ist somit eine einfache, großtechnisch leicht einsetzbare Regenerierung des Hydrolyseaktivators möglich.

Durch Parallelschalten von mindestens zwei mit Hydrolyseaktivator gefüllten Reaktoren wird zudem eine kontinuierliche Verfahrensführung möglich, wobei ein Reaktor im Dechloriermodus und ein weiterer im Regeneriermodus betrieben wird.

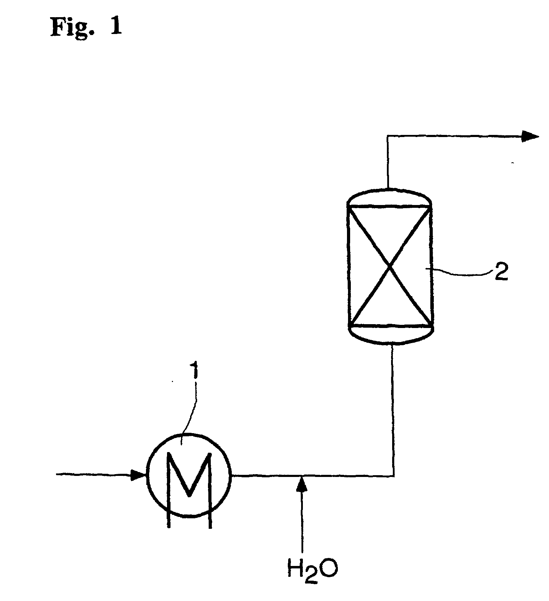

## Patentansprüche

1. Verfahren zur Hydrolyse von Alkylmonohalogeniden aus einem diese enthaltenden Gemisch in Gegenwart von Hydroxiden und Aktivkohle, **dadurch gekennzeichnet, daß** die Hydrolyse an mit Alkyli- und/oder Erdalkalihydroxiden imprägnierter Aktivkohle in Gegenwart von Wasser durchgeführt wird.

2. Verfahren nach Anspruch 1, **dadurch gekennzeichnet, daß** das Gemisch mindestens eines der C1- bis C4-Alkylmonohalogenide enthält

3. Verfahren nach Anspruch 1 oder 2, **dadurch gekennzeichnet, daß** die Aktivkohle mit 1 bis 40 Gew.-% Alkali- und/oder Erdalkalihydroxid imprägniert ist.

4. Verfahren nach einem der Anprüche 1 bis 3, **dadurch gekennzeichnet, daß** die Aktivkohle mit 5 bis 25 Gew.-% Alkali- und/oder Erdalkalihydroxid imprägniert ist.

5. Verfahren nach Anspruch 3 oder 4, **dadurch gekennzeichnet, daß** Natriumhydroxid verwendet wird.

6. Verfahren nach einem der vorhergehenden Ansprüche, **dadurch gekennzeichnet, daß** in einem Temperaturbereich von Raumtemperatur bis 600°C gearbeitet wird.

7. Verfahren nach einem der vorhergehenden Ansprüche, **dadurch gekennzeichnet, daß** in einem Temperaturbereich von 120 bis 300°C gearbeitet wird.

8. Verfahren nach einem der vorhergehenden Ansprüche, **dadurch gekennzeichnet, daß** bei einem Druck von 1,5 bar a bis 50 bar a gearbeitet wird.

9. Verfahren nach eine der vorhergehenden Ansprüche, **dadurch gekennzeichnet, daß** bei einem Druck von 1 bis 5 bar a gearbeitet wird.

10. Verfahren nach einem der vorhergehenden Ansprüche, **dadurch gekennzeichnet, daß** es in der Gasphase durchgeführt wird.

11. Verfahren nach einem der Ansprüche 1 bis 6, **dadurch gekennzeichnet, daß** es in flüssiger Phase durchgeführt wird.

12. Verwendung des Verfahrens nach einem der vorhergehenden Ansprüche als Darstellungsmethode für Alkohole.

## Claims

1. A process for hydrolyzing alkyl monohalides in a mixture comprising same in the presence of hydroxides and activated carbon, which comprises conducting the hydrolysis in the presence of water over activated carbon impregnated with alkali metal hydroxide or alkaline earth metal hydroxide.

2. A process as claimed in claim 1, wherein the mixture comprises at least one C₁-C₄-alkyl monohalide.

3. A process as claimed in claim 1 or 2, wherein the activated carbon has been impregnated with from 1 to 40% by weight of alkali metal hydroxide or alkaline earth metal hydroxide.

4. A process as claimed in any of claims 1 to 3, wherein the activated carbon has been impregnated with from 5 to 25% by weight of alkali metal hydroxide or alkaline earth metal hydroxide.

5. A process as claimed in claim 3 or 4, wherein sodium hydroxide is used.

6. A process as claimed in any of the preceding claims, wherein the temperature is in the range from room temperature to 600°C.

7. A process as claimed in any of the preceding claims, wherein the temperature is in the range from 120 to 300°C.

8. The process as claimed in any of the preceding claims, wherein the pressure is in the range from 1.5 bar absolute to 50 bar absolute.

9. A process as claimed in any of the preceding claims, wherein the pressure is in the range from 1 to 5 bar absolute.

10. A process as claimed in any of the preceding claims, conducted in the gas phase.

11. A process as claimed in any of claims 1 to 6, conducted in the liquid phase.

12. The use of the process of any of the preceding claims for preparing alcohols.

## Revendications

1. Procédé pour l'hydrolyse de monohalogénures d'alkyle à partir d'un mélange les contenant en présence d'hydroxydes et de charbon actif, **caractérisé en ce que** l'hydrolyse est réalisée sur du charbon actif imprégné d'hydroxydes alcalins et/ou de métaux alcalino-terreux en présence d'eau.

2. Procédé selon la revendication 1, **caractérisé en ce que** le mélange contient au moins un des monohalogénures d'alkyle en C₁ à C₄.

3. Procédé selon la revendication 1 ou 2, **caractérisé en ce que** le charbon actif est imprégné de 1 à 40 % en poids d'hydroxyde alcalin et/ou de métal alcalino-terreux.

4. Procédé selon l'une quelconque des revendications 1 à 3, **caractérisé en ce que** le charbon actif est imprégné de 5 à 25 % en poids d'hydroxyde alcalin et/ou de métal alcalino-terreux.

5. Procédé selon la revendication 3 ou 4, **caractérisé en ce qu'**on utilise de l'hydroxyde de sodium.

6. Procédé selon l'une quelconque des revendications précédentes, **caractérisé en ce qu'**on travaille dans une plage de température allant de la température ambiante à 600 °C.

7. Procédé selon l'une quelconque des revendications précédentes, **caractérisé en ce qu'**on travaille dans une plage de température allant de 120 à 300 °C.

8. Procédé selon l'une quelconque des revendications précédentes, **caractérisé en ce qu'**on travaille à une pression de 1,5 bars abs à 50 bars abs.

9. Procédé selon l'une quelconque des revendications précédentes, **caractérisé en ce qu'**on travaille à une pression de 1 à 5 bars abs.

10. Procédé selon l'une quelconque des revendications précédentes, **caractérisé en ce qu'**il est réalisé en phase gazeuse.

11. Procédé selon l'une quelconque des revendications 1 à 6, **caractérisé en ce qu'**il est réalisé en phase liquide.

12. Utilisation du procédé selon l'une quelconque des revendications précédentes comme méthode de préparation d'alcools.
